# EUROPEAN PATENT SPECIFICATION

(11) **EP 4 111 972 B1**
(45) Date of publication and mention of the grant of the patent: **22.01.2025**
(21) Application number: 21461563.5
(22) Date of filing: 30.06.2021
(51) Int. Cl.: A61B 5/25, A61B 5/257, A61B 5/271, A61B 5/282

(54) **ELECTRODE SEGMENT AND ELECTRODE MATRIX FOR ELECTROCARDIOGRAPHIC AND/OR BIOIMPEDANCE MEASUREMENTS**
ELEKTRODENSEGMENT UND ELEKTRODENMATRIX FÜR ELEKTROKARDIOGRAPHISCHE UND/ODER BIOIMPEDANZMESSUNGEN
SEGMENT D'ÉLECTRODES ET MATRICE D'ÉLECTRODES POUR LES MESURES ÉLECTROCARDIOGRAPHIQUES ET/OU DE BIOIMPÉDANCE

(43) Date of publication of application: 04.01.2023
(73) Proprietor: Wojskowa Akademia Techniczna, 00-908 Warszawa (PL); Wojskowy Instytut Medyczny, 04-141 Warszawa (PL)
(72) Inventor: WALCZAK, Andrzej, 00-908 Warszawa (PL); MARCINIAK, Piotr, 00-908 Warszawa (PL); BACZYK, Piotr, 00-908 Warszawa (PL); KRZESINSKI, Pawel, 04-141 Warszawa (PL); MURAWSKI, Piotr, 04-141 Warszawa (PL)
(74) Representative: Wlasienko, Jozef

(56) References cited:
- EP-A2- 1 275 342
- AU-A1- 2017 279 796
- GB-A- 2 527 031
- US-A- 6 129 666
- US-A1- 2021 113 109
- B. G. LAPATKI: "A thin, flexible multielectrode grid for high-density surface EMG", JOURNAL OF APPLIED PHYSIOLOGY, vol. 96, no. 1, 29 August 2003 (2003-08-29), US, pages 327 - 336, XP055298688, ISSN: 8750-7587, DOI: 10.1152/japplphysiol.00521.2003

## Description

### Field of the Invention

The object of the present invention is an electrode segment and an electrode array for electrocardiographic and/or bioimpedance measurements. The electrode segment and the electrode array according to the invention are in principle disposable and may be used in a hospital and/or home conditions. The invention may find application in particular for impedance cardiography measurements.

### Background of the Invention

Nowadays, cardiovascular diseases are one of the leading causes of death worldwide, especially in the EU countries. Bioimpedance methods such as impedance cardiography (ICG) and electrocardiography are the primary methods for the early detection and diagnosis of cardiovascular diseases. The primary measuring sensor in these methods are electrodes, which, depending on the method, are arranged in different places on the patient's body. Consequently, the patient's anatomical features significantly affect the accuracy of the electrode contact with the patient's skin. This is particularly important in the case of impedance cardiography measurements, in the case of which the electrodes are arranged at the base of the neck and on the midaxillary line of the xiphoid process.

Due to ergonomics and hygiene rules in hospital and home conditions, disposable electrodes are used to carry out measurements. These electrodes usually consist of a polymeric support layer, in which the electrode is embedded, and which is covered with an adhesive layer. The adhesive layer is used to stick the electrodes to the patient's body. However, differences in anatomical structure and physiological effects such as the presence of sweat on the surface of the patient's skin, in combination with the substantially flat, solid support layer of the electrodes currently available, causes detachment of the electrodes. Deterioration or complete lack of contact of the electrode with the patient's skin during the measurement causes distortion or disturbance of the measured signal, thus leading to erroneous interpretation of the measurement results or the necessity to repeat the measurements.

### State of the Art

Various electrode systems used for bioimpedance and electrocardiographic measurements are known in the art. The problem of adequate attachment of disposable electrode systems, especially those containing more than one electrode, has been attempted, for example, in the application WO2018193271A1, which discloses an electrocardiographic sensor comprising an electrode array, which comprises a substrate connecting at least three electrodes spaced apart from each other, and a flexible sheet with a larger area than the surface area of the electrode array and configured to attach the electrodes to the patient's body. The use of a flexible sheet, in addition to the substrate for the electrodes, provides an additional area of contact with patient's skin, due to the surface tension between the sheet and patient's skin, to attach the electrode to the skin without the need for an adhesive use. This sensor can be applied and removed without any discomfort to the patient. However, to ensure proper adherence of the electrodes to the patient's skin, the flexible sheet must have a substantially larger surface area than the electrode array, and moreover, preferably the electrode array and flexible sheet should be applied on flat areas of the patient's body and preferably in a recumbent, resting position. The invention therefore cannot be easily applied to various leads in electrocardiographic and bioimpedance methods, which may be located on surfaces that are substantially non-planar and anatomically diverse, such as for example the extremities or neck of a patient, and the measurements have to be made under different conditions of patient's position and motion.

In the application GB2527031A, the use of large-area adhesive means was abandoned in favour of localized adhesive means. The application discloses an electrode array comprising a flexible backing material in which round electrodes are situated, each immediately surrounded by an annular adhesive layer. The electrode array is preferably "X"-shaped, with the electrodes positioned at the ends of the arms, and a reference electrode may be provided in the central portion. To improve flexibility of the electrode array arms, the flexible backing material is provided with a perforation which may take various shapes and positions but is disposed substantially between the end electrodes and the central electrode. Such perforation may facilitate adaptation of the electrode array to the patient's body, but will not prevent the possibility of individual and/or even all electrodes becoming detached. The ring of the adhesive layer immediately surrounding the electrode will be subjected to peel stresses resulting from the direct contact between the rigid electrode surface and unevenness of the patient's body parts, as well as structural stresses of a backing material that is substantially not attached to the patient's skin. Moreover, the ring surrounding the electrode must have substantially the same thickness as the electrode, causing additional problems in the manufacture of the electrode array. With a greater thickness of the adhesive ring, the sufficient contact of the electrode with the patient's skin will not be ensured, while with a smaller thickness, the electrode will detach due to stresses between the electrode and the patient's skin.

In both of the above cases, the electrodes immediately surrounded by the adhesive layer create a significant surface area covering the skin without access to air and adequate ventilation, which can lead to excessive sweating and deterioration of the prior art electrode adherence.

Furthermore, in the state of the art the publication titled: "A thin, flexible multielectrode grid for high-density surface EMG" JOURNAL OF APPLIED PHYSIOLOGY, vol. 96, no. 1, 29 August 2003 (2003-08-29), pages 327-336, XP055298688, US ISSN: 8750-7587, DOI: 10.1152/japplphysio1.00521.2003 is known, in which discloses a thin, flexible electrode grid for the high density surface EMG. The grid is made of thin, electrode carrying material. Additionally, to facilitate the skin attachment, some perforation orifices are made. Both electrodes and perforation orifices are arranged regularly within the grid, with the same distance from each other. In order to attach the grid to a patient skin double sided medical tape is provided with orifices corresponding both to perforation orifices of the grid and the electrodes. In this way the perforation orifice is not covered by the adhesive tape and the electrodes are provided with the skin contact, matching the electrode orifices in the adhesive tape. As a result, when the grid is placed on the adhesive tape the whole carrying material of the grid is covered by the adhesive layer.

In a prior art according to the solution there is no perforated area provided in such the assembled grid, which is free from the adhesive layer and that surrounds measuring electrode, but only perforation itself (as perforation orifice) that is arranged in four corners in a certain distance from the measuring electrode. Moreover, the electrode in this case is surrounded by the adhesive area which is different from the claimed solution of the invention. In the solution according to the invention the whole perforated area (amounting layer and the hole provided within) must be free from adhesive and not only the hole (perforation) itself.

In the state of the art, there is also known US patent No. US 6 129 666 entitled *"Biomedical electrode",* where a biomedical electrode including a flexible pad having a top surface and a bottom surface; an asymmetrical, linearly aligned array of signal contacts retained by the flexible pad and each having a contact surface projecting from the bottom surface and a coupling surface projecting above the top surface; and a connector including a plurality of connector contacts each being shaped and arranged for electrical connection to a different one of the coupling surfaces. The asymmetrical array of signal contacts facilitates proper positioning of the electrode on the skin.

In a similar way as in document above-mentioned, in this case openings are also arranged within the area which is part of adhesive surface. Also when adhesive surface is placed on a moulded case, the contacts are surrounded by the adhesive area of the surface and not by the perforated area which is free from the adhesive layer.

Technical problems that have not been solved so far in the prior art are, in particular:
- structure of electrode arrays causing peel stress in the immediate vicinity of the measuring electrode, especially in areas with variable shape and unevenness;
- large adhering surface causing problems when sticking the electrode to varying shapes of the patient's body or too small adhering surface causing insufficient electrode contact;
- lack of scalability and structural versatility, as well as the need to ensure the precision of shape and selection of materials to achieve the intended adhesion effect, which leads to applications for strictly dedicated measurement methods.

The present invention in the form of an electrode segment and an electrode array for electrocardiographic and/or bioimpedance measurements of the claimed structure and application solves the above technical problems.

### Summary

The object of the present invention is to ensure a completely new solution that provides an electrode segment and an electrode array for electrocardiographic and/or bioimpedance measurements.

In accordance with one aspect of the present invention, the present invention provides an electrode segment for electrocardiographic and/or bioimpedance measurements comprising a measuring electrode, which comprises a connector element for connecting an electrical signal wire and a sensor element for collecting measurement signals from the patient's body, the measuring electrode being situated in a mounting layer covered with an adhesive layer, wherein the measuring electrode situated in the mounting layer is surrounded by a perforated area which is free from the adhesive layer, and the perforated area is in turn surrounded by a surface-continuous area which is covered with the adhesive layer, wherein the perforated area comprises radially arranged alternating void spaces and solid spaces.

Preferably, the measuring electrode is situated substantially centrally in the perforated area.

Preferably, the perforated area is substantially round in shape.

Preferably, the solid spaces and the void spaces are elongated in shape and are arranged radially from the measuring electrode to the surface-continuous area.

Preferably, the surface-continuous area, which is covered with the adhesive layer is substantially annular in shape.

Preferably, the mounting layer and the adhesive layer are flexible layers configured to adapt their shape to the shape of the substrate to which they are being attached.

Preferably, the thickness of the adhesive layer is smaller than the thickness of the sensor element of the measuring electrode.

Preferably, the connector element is a snap-fit socket for the electrical signal wire.

In accordance with another aspect of the present invention, the present invention provides an electrode array for electrocardiographic and/or bioimpedance measurements, characterized in that the array comprises at least two electrode segments according to the invention, and the electrode segments are connected to each other by a connecting area which is adjacent to the particular electrode segments.

Preferably, the connecting area forms a uniform layer with the surface-continuous area of the electrode segment and is covered with the adhesive layer.

Preferably, the connecting area is substantially centrally situated and surrounded by the electrode segments.

Preferably, the electrode segments connected by the connecting area are arranged such that each electrode segment is situated substantially equidistantly from the adjacent electrode segment.

Preferably, electrode array has a multi-segment configuration with free bending of the particular electrode segments configured to adapt their shape to the shape of the substrate to which they are being adhered.

Preferably, the connector elements of the measuring electrode are electrically connected in parallel such that each measuring electrode has a branch of the same electrical signal wire measures the same electric potential at each measuring electrode.

### Detailed Description of the Invention

An electrode segment for electrocardiographic and/or bioimpedance measurements according to the invention comprises a measuring electrode, which comprises a connector element for connecting an electrical signal wire and a sensor element for collecting measurement signals from the patient's body. Measuring electrodes used for measurements in such segments comprise a substantially circular and substantially flat sensor element, one side of which is intended to come into contact with the patient's skin, and the connector element of which is arranged on the opposite, outer side of the sensor element.

In principle, such electrodes are well known in the art as well as commercially available, and any medically approved electrode may be used within the scope of the invention. The connector element may be any required connector element that is compatible with the electrical signal wire connections of the electrocardiographic and bioimpedance measuring device. Preferably, the connector element is a snap-fit socket for the electrical signal wire.

The connector element may be arranged substantially arbitrarily, but preferably the connector element is arranged in a central part of the outer side of the sensor element of the measuring electrode and its diameter is smaller than the diameter of the sensor element of the measuring electrode, and more preferably the diameter of the connector element is at least two times smaller than that of the sensor element of the measuring electrode. Preferably, the diameter of the electrode is from 5 mm to 9 mm. Preferably, the diameter of the connector element is suitably 2.5 mm. The electrode may preferably be made of a conductive material, preferably a corrosion-resistant one. Preferably, the electrode is made of a corrosion-resistant metal. The electrode is preferably made of duralumin, more preferably of PA9 zinc duralumin. Preferably, the material of the measuring electrode has a tensile strength of 350 MPa to 450 MPa, more preferably 400 MPa to 450 MPa.

According to the invention, the electrode segment for electrocardiographic and bioimpedance measurements further comprises a mounting layer and an adhesive layer. It should be understood within the scope of the present description that both the mounting layer and the adhesive layer may be surface-discontinuous.

Preferably, the measuring electrode is situated in the mounting layer such that the sensor element of the measuring electrode is arranged on the first side of the mounting layer, which is the side applied to the patient's skin and to which the adhesive layer is applied, and the connector element is arranged on the other side of the mounting layer, which is the outer side of the electrode segment.

According to the invention, the electrode segment for electrocardiographic and bioimpedance measurements is characterized in that the measuring electrode situated in the mounting layer is surrounded by a perforated area which is free from an adhesive layer, and the perforated area is in turn surrounded by a surface-continuous area which is covered with an adhesive layer. This should be understood as meaning that, in the electrode segment according to the invention, the mounting layer comprises a perforated area which is free from the adhesive layer and directly adjoins the measuring electrode and surrounds it circumferentially, and comprises a surface-continuous area which is covered with an adhesive layer and directly adjoins the perforated area and surrounds it circumferentially.

Such a structure of the electrode segment according to the invention reduces the peel stress in the immediate vicinity of the electrode. This is due to the provision of a perforated area between the sensor element of the measuring electrode and the adhesive layer, which is more flexible and can react by deformation to the unevenness of the substrate to which the electrode is attached. In addition, the perforated area provides ventilation of the patient's skin in the area of the electrode segment attachment, preventing excessive sweating and a negative impact on the adherence of the adhesive layer.

The measuring electrode may be situated substantially arbitrarily in the perforated area, but preferably the measuring electrode is situated substantially centrally in the perforated area. The central location of the electrode in the perforated area enables the most uniform distribution of stresses resulting from the contact of both the measuring electrode and the entire electrode segment with the substrate.

The perforated area surrounding the measuring electrode may be of any shape. Preferably, the perforated area is substantially round in shape. The perforated area surrounding circumferentially the measuring electrode has an area preferably larger than that of the measuring electrode itself, and more precisely larger than that of the sensor element of the measuring electrode. Preferably, the diameter of the perforated area is 1.5 to 2 times the diameter of the surface of the sensor element. Preferably, the diameter of the perforated area is at least 1.5 or 1.6 or 1.7 or 1.8 or 1.9 or 2 times the diameter of the surface of the sensor element. Preferably, for a sensor element with a diameter of 5 mm, the diameter of the perforated area is equal 9 mm. A too small diameter of the perforated area can insufficiently reduce the peel stress, while an excessively large diameter of the perforated area may not ensure sufficient contact of the electrode with the substrate. Considering the above, person skilled in the art will readily select the dimensions of the perforated area according to the dimensions (diameter and thickness) of the electrode and the requirements of the measurement method.

Preferably, the perforated area is formed by solid spaces and void spaces. As used herein, this should be understood as meaning that the void spaces may otherwise be referred to as perforation holes and the solid spaces are the area remaining in the perforated area after the perforation areas have been cut.

The solid spaces and the void spaces can be substantially of any area and any shape of any dimension within the perforated area. Depending on the required degree of flexibility, one can choose the area and shape of any size of solid spaces and void spaces. Preferably, the solid spaces and the void spaces are elongated in shape and are arranged radially from the measuring electrode to the surface-continuous area.

Considering the above, one skilled in the art will readily select the dimensions and shape of the solid spaces and void spaces to obtain the desired flexibility of the perforated area to obtain the best fit of the electrode segment to the measurement substrate while maintaining sufficient adherence of the adhesive layer and the contact between the electrode and the measurement substrate.

The perforated area is surrounded by the surface-continuous area. The surface-continuous area can have substantially any shape and dimensions. Preferably, the surface-continuous area, which is covered with the adhesive layer is substantially annular in shape. The inner diameter of the surface-continuous area is substantially equal to the diameter of the perforated area it surrounds. The width of the surface-continuous area that is covered with the adhesive layer, i.e. in the solution presented herein also the width of the adhesive layer of the electrode segment, must ensure adherence of the electrode segment. Preferably, the width of the surface-continuous area is greater than or equal to 1 mm, more preferably the width of the surface-continuous area is greater than or equal to 3 mm.

In other words, preferably the surface-continuous area covered with the adhesive layer forms the outer envelope, from which to the center of this layer, mutually noncontacting linking elements leading to the inner closed strip surrounding the central opening in the center of the mounting layer are led out, forming a perforated area, the measuring electrode being arranged above the central opening and preferably the connector element of the measuring electrode is passed through the central opening.

As indicated above, the perforated area as well as the surface-continuous area may have any shape, such as oval, triangular or square, preferably with rounded corners, or the shape of an irregular flat figure. One skilled in the art will readily select the appropriate shape for the measurement method and for the shape of the surface to which the electrode segment is to be attached. However, as indicated above, a round shape is preferred.

Regardless of the above-mentioned changes in the flexibility of the mounting layer areas through the use of a suitable perforated area, preferably the mounting layer and the adhesive layer are flexible layers configured to adapt their shape to the shape of the substrate to which they are attached. The mounting layer can be made of any material, preferably a polymer, that is intended for medical applications, especially in the field of electrocardiographic and bioimpedance measurements, such as polyimide, polyethylene foam, polyester non-woven fabric and the like.

Preferably, the modulus of elasticity of the surface-continuous area of the support layer, i.e. the support layer without perforation, is from 3 GPa to 4 GPa. Preferably, the thickness of the surface-continuous area of the support layer and the solid spaces of the perforated area of the support layer is from 0.5 mm to 0.7 mm. Preferably, the mounting layer is made of polyimide.

The thickness of the adhesive layer is in principle less than the thickness of the support layer, and preferably the ratio of the thickness of the adhesive layer to the thickness of the support layer is 4:1. Preferably, the thickness of the adhesive layer ranges from 0.1 mm to 0.2 mm.

Preferably, the thickness of the adhesive layer is smaller than the thickness of the sensor element of the measuring electrode. The thickness of the sensor element of the measuring electrode for bioimpedance measurements is preferably 1.1 to 10 times the thickness of the adhesive layer. However, also for a wider range of thicknesses of the sensor element of the measuring electrode, the introduction of a perforated area surrounding the sensor element of the measuring electrode enables good contact of the electrode with the substrate by means of the electrode segment according to the invention. A person skilled in the art will readily select the relative thicknesses of the adhesive layer and the sensor element depending on the material of the adhesive layer, the leads required, and the parts of the patient's body to which the electrode segment is to be attached.

Adhesive layers such as, for example, adhesives, hydrogels for disposable electrodes for electrocardiographic or bioimpedance measurements, are well known to those skilled in the art. The adhesive layers selected for disposable electrodes should be characterized by good adherence to the patient's skin, but at the same time allow for the removal of the disposable electrode without the use of a solvent. Such adhesive layers can be arbitrarily chosen depending on the required thickness and the thickness of the sensor element of the measuring electrode and the material of the support layer. The adhesive layer can be applied, for example, in the form of a glue layer, a gel adhesive layer, a polymer layer coated on both sides with an adhesive. An adhesive layer comprising a polymer layer coated on both sides with an adhesive is particularly preferred. By selecting the thickness of the polymer layer, an adhesive layer of a given thickness can be obtained much easier than with a hydrogel. Moreover, this type of adhesive layer makes it easier to correlate the thickness of the sensor element and the thickness of the adhesive layer. A medical grade acrylic adhesive and a thin polyimide layer with a thickness of preferably about 0.2 mm to 0.1 mm may preferably be used to form the adhesive layer.

In view of the above, it is preferable to consider as the adhesive layer each element of the electrode segment that is arranged on the first side of the mounting layer, circumferentially surrounds the perforated area of the mounting layer and projects above the perforated area, and exhibits adherence.

Moreover, preferably at least the adhesive layer, more preferably the electrode segment, in a state before use may be covered with a protective layer such as, for example, a paper layer or a layer of other suitable material known to those skilled in the art to protect the adhesive layers.
For example, an electrode segment can be manufactured by a method comprising the following steps:
- cutting a predetermined shape of the mounting layer of the electrode segment out from the polymer sheet;
- coating or applying an adhesive layer to the mounting layer.
Alternatively, the manufacturing method comprises:
- coating or applying an adhesive layer to the polymer sheet;
- cutting a predetermined shape of the mounting layer of the electrode segment out from the polymer sheet.

Furthermore, the electrode segment according to the invention can be manufactured by any method known to a person skilled in the art to obtain a planar element, preferably a polymeric mounting layer, such as the solution casting with solvent evaporation or 3D printing, for example.

One of the technical problems identified in the prior art is the lack of scalability and structural versatility of existing electrode systems for electrocardiographic and bioimpedance measurements. This problem can be solved by another aspect of the present invention which is the electrode array. The electrode segment according to the invention makes it possible to create an electrode array that comprises at least two electrode segments according to the invention and at the same time does not lose the ability to adapt easily to the measurement substrate. The electrode segments according to the invention forming the electrode array are connected to each other by a connecting area which is adjacent to particular electrode segments. In principle, by such a connection, it is possible to predefine the necessary number of electrode segments required for carrying out the corresponding cardiographic and/or bioimpedance measurements. In principle, the connecting element may have any shape to match the final shape of the required electrode array. The connecting element can be made of any material depending on the purpose and desired function, and a person skilled in the art will readily select the shape, material and method of connecting the electrode segments according to the invention to form the electrode array according to the invention.

Preferably, the connecting area forms a uniform layer with the surface-continuous area of the electrode segment and is covered with the adhesive layer. This reduces the number of electrode array layers according to the invention and greatly simplifies the manufacturing method the electrode array, since the entire array can be manufactured as a uniform support layer and coated with an adhesive layer, with only the perforated areas free of the adhesive layer. As a result, adhering surfaces on the measurement surface, i.e. patient's skin, are additionally obtained through the adhesive layer on the connecting element.

The electrode array can take substantially any shape based on electrode segments that can be joined by at least one connecting element. For example, the electrode array may be elongated in shape by connecting at least two electrode segments, or three electrode segments, or four electrode segments, or five electrode segments, or six electrode segments, by means of one connecting element, or two connecting elements, or three connecting elements, or four connecting elements, or five connecting elements, respectively. Preferably, however, the connecting area is situated substantially centrally and is surrounded by the electrode segments.

Moreover, to reduce the surface area of the electrode array, preferably, the electrode segments connected by the connecting area are arranged such that each electrode segment is situated substantially equidistantly from the adjacent electrode segment. This is the way to create the most compact electrode array. Preferably, it may be a combination of three electrode segments, where the electrode segments are arranged at the corners of an isosceles triangle. Preferably, it may be a combination of four electrode segments, where the electrode segments are arranged at the corners of the square. The distances between the particular electrode segments can be suitably adjusted as required, but preferably the adhesive layers of the individual electrode segments are directly adjacent to each other.

Additionally, preferably, electrode array has a multi-segment configuration with free bending of the particular electrode segments configured to adapt their shape to the shape of the substrate to which they are being adhered. This should be understood as meaning that preferably the adjacent electrode segments are in principle connected to the connecting area at only one point of the surface-continuous area and/or the adhesive layer of each electrode segment, and the size of the connecting area at the connection point to each electrode segment constituting the electrode array is substantially smaller than the diameter of the electrode segment. Preferably, the size of the connecting area at the connection point with each electrode segment constituting the electrode array is less than 0.5 times the diameter of the electrode segment, more preferably less than 0.33 times the diameter of the electrode segment, even more preferably 0.25 times the diameter of the electrode segment. For example, in the case where the electrode array is formed by three electrode segments, the electrode segments are preferably connected by "Y", "T" connecting area to a substantially triangular shape, such that the segments are arranged at the ends of the arms and at the base of the "Y". On the other hand, in the case where the electrode array is formed by four electrode segments, the electrode segments are preferably connected by an "X" - shaped connecting area, such that the segments are arranged at the ends of the arms and at the base of the "X". A person skilled in the art will select without undue burden the shape of the connecting area to the desired shape of the electrode array.

Another problem indicated in the prior art is the deterioration or complete loss of contact of the electrode with the patient's skin during the measurement due to detachment of the electrode, which causes distortion or disturbance of the measured signal, thus leading to misinterpretation of the measurement results or the need to repeat the measurements. Although the structure of the electrode segment according to the invention significantly reduces the risk of loss or deterioration of contact by a single electrode, but also in this case undesirable disturbances in the measurements can occur. In the electrode array according to the invention, each electrode segment is provided with an independent connector element. This means that within the electrode array, each segment can be freely connected in accordance with the accepted requirements. In order to compensate for measurement errors resulting from a reading from a single electrode segment, the electrode segments provided with measuring electrodes preferably comprise connector elements electrically connected in parallel, so that each measuring electrode has a branch of the same electrical signal wire which measures the same electric potential at each measuring electrode. This results in much greater measurement stability. Preferably, the connector element is a snap-fit element.

Moreover, as used herein, the term "diameter" is intended to mean the diameter of a set, which is the supremum of the distance of all pairs of points for a limited subset of a metric space, that is, of any figure or section. Unless otherwise stated, when all quantities are defined by numerical values and ranges, ranges resulting from any combination of their values provided herein and values falling within the given ranges are also preferred.

The solution provided according to the technical features proposed above enables:
- minimizing the peel stress in the immediate vicinity of the measuring electrode, especially in areas of variable shape and unevenness, such as the patient's neck, with anatomical variations such as, for example, a short neck, a wide neck, a slim neck with a deep "clavicular fossa", etc.;
- optimizing the area of the adherence surface to reduce problems with sticking the electrode to the changing shapes of the patient's body;
- obtaining greater measurement reliability due to the structural features of both the electrode segment and the electrode array, including the possibility of selecting the electrical connection, especially the parallel connection of the electrode segments;
- obtaining scalability and structural versatility, not only through the possibility of combining the segments into an electrode array and selecting the type of connections, but also easily modifying the properties of the system through the interaction of material features and shape features of the electrode segment and the electrode array.

For example, it is worth noting that diversified flexibility is obtained when using a single support layer without the need to change the material within this layer by using a perforated area with a structure selected as required and with the sizes of particular areas of the electrode segment and the electrode array. The layer may be cut out from a sheet of the selected polymer and cut off to the desired shape.

### Brief description of the drawing

The object of the present invention is disclosed in the embodiments with reference to the attached drawing, in which:
- FIG. 1A: is a top view of an electrode segment;
- FIG. 1B: is a bottom oblique view of an electrode segment;
- FIG. 2A: is a top oblique view of an electrode array comprising four electrode segments;
- FIG. 2B: is a bottom oblique view of an electrode array comprising four electrode segments;
- FIG. 3: is a top view of an electrode array comprising two electrode segments;
- FIG. 4A: is a top view of an electrode array comprising three electrode segments;
- FIG. 4B: is another top view of an electrode array comprising three electrode segments.

### Embodiments of the Invention

Herein below, object of the present invention is described in detail with reference to the accompanying Figures and embodiments. Figures are illustrative of the invention and have been made without precisely keeping to the scale and aspect ratio of the elements depicted therein. The present invention is not limited to the specific embodiments described herein.

The following may be used to implement the following embodiments according to the invention:
- as the mounting layer: polyimide, in the form of a flexible film with a thickness of 0.7 mm
- as the adhesive layer: polyimide, in the form of a flexible film with a thickness of 0.2 mm, coated on both sides with medical-grade acrylic adhesive
- as the measuring electrode: the standard electrode used to manufacture disposable electrodes for ECG or ICG measurements, 5 mm in diameter and 1 mm thick.

### First Embodiment of the Invention

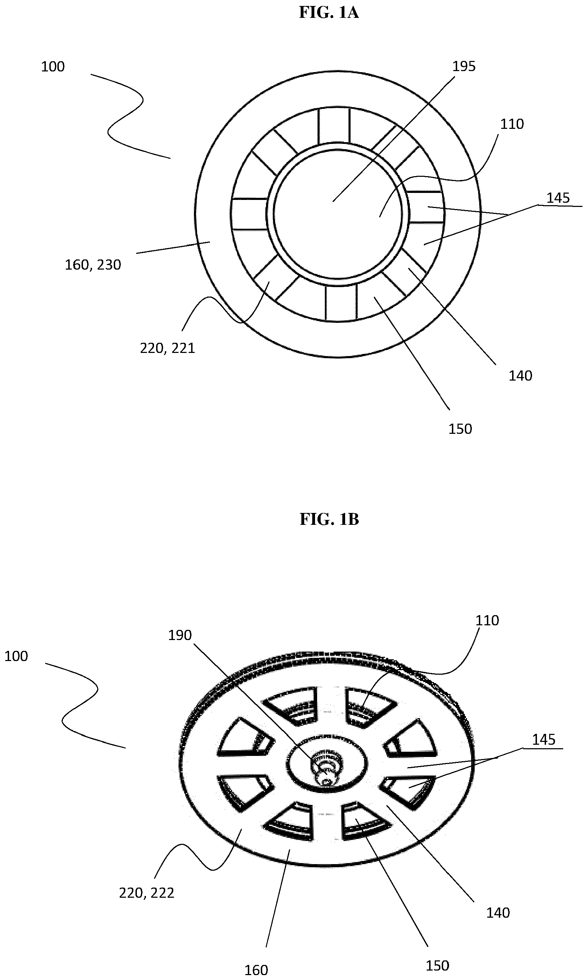

In the first embodiment of the invention, the electrode segment 100 according to the invention is shown. Fig. 1A and 1B are top and oblique bottom views, respectively, for the electrode segment 100 according to the invention.

The electrode segment 100 was manufactured by cutting out the desired circular shape of the mounting layer 220, along with the perforated area 145 comprising radially arranged alternating void spaces 150 and solid spaces 140. Thereafter, the adhesive layer 230 was applied to the first side 221 of the mounting layer 220 with the surface-continuous area 160 shape, in the shape of a circular ring. The measuring electrode 110 is arranged in the center of the perforated area 145 such that the sensor element 195 of the measuring electrode 110 is on the first side 221 of the mounting layer 220 and the connector element 190 is on the other side 222 of the mounting layer 220, as shown in Fig. 1A and Fig. 1B.

Thus, the circular electrode segment with a diameter of 15 mm was obtained, the width of the surface-continuous area 160 of the circular ring shape covered with the adhesive layer 230 being 3 mm.

### Second Embodiment of the Invention

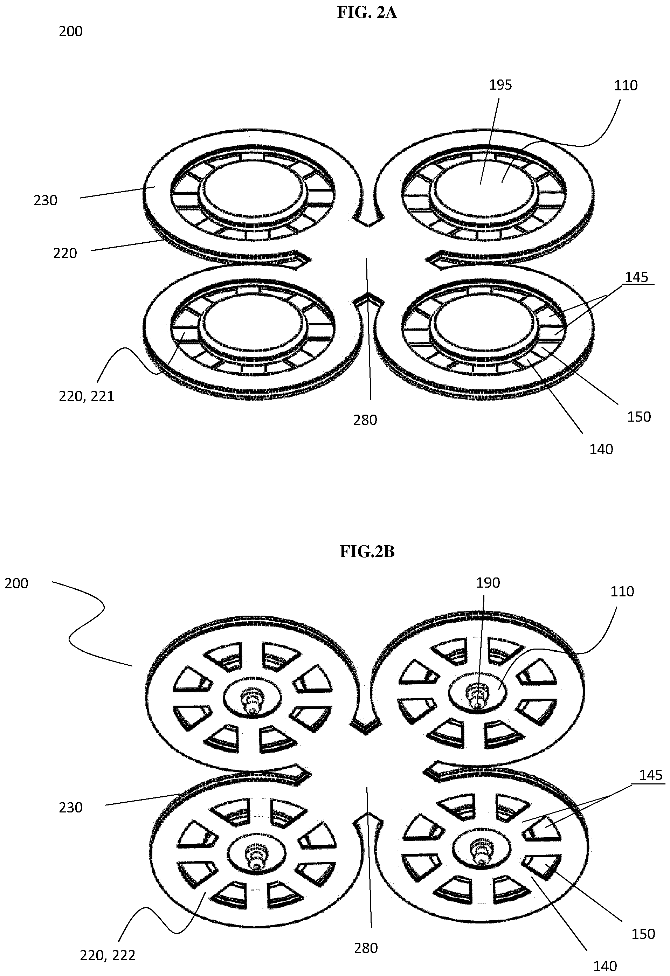

In the second embodiment of the invention, the electrode array 200 according to the invention is shown. Fig. 2A and 2B are top oblique and bottom oblique views, respectively, for the electrode array 200 according to the invention comprising four electrode segments 100 according to the invention.

The electrode array 200 was made by cutting out a desired shape reminding the "four-leaf clover" of the mounting layer 220 along with the perforated area 145 comprising radially alternating void spaces 150 and solid spaces 140. The mounting layer 220 now comprises the X-shaped connecting area 280, such that the electrode segments 100 are connected by the connecting area 280, and are disposed such that each electrode segment 100 is situated equidistantly from an adjacent electrode segment 100. In other words, the connecting area 280 directly adjoins the surface-continuous area 160. Next, the adhesive layer 230 having the surface-continuous area 160 of the electrode segments 100 shape along with the connecting area 280 shape is applied to the first side 221 of the mounting layer 220. The measuring electrodes 110 are positioned in the center of the perforated areas 145 of each electrode segment 100, such that the sensor element 195 of the measuring electrode 110 is provided on the first side 221 of the mounting layer 220 and the connector element 190 is on the other side 222 of the mounting layer 220, as shown in Fig. 2A and Fig. 2B.

### Third Embodiment of the Invention

The electrode array 200 of the third embodiment is shown in Fig. 2A and Fig. 2B, and is the same as the electrode array 200 of the second embodiment, except that according to the third embodiment, connection wires (not shown) were electrically connected in parallel to the connector elements 190 in the form of a snap-fit element such as shown in Fig. 2B, such that each measuring electrode has a branch of the same electrical signal wire, which measures the same electric potential at each measuring electrode.

### Fourth Embodiment of the Invention

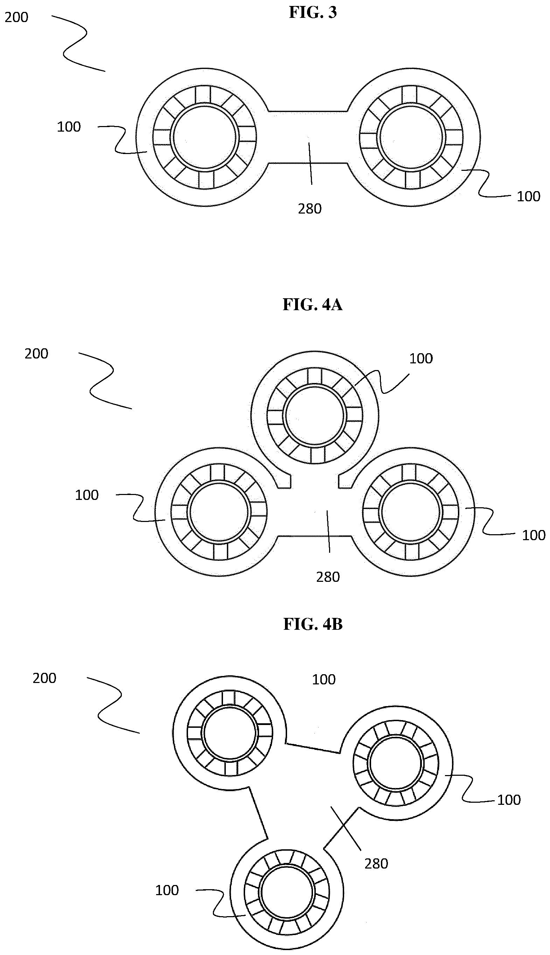

In the fourth embodiment of the invention, another electrode array 200 according to the invention is shown. Fig. 3 is a top view for the electrode array 200 according to the invention comprising two electrode segments 100 according to the invention. The electrode array 200 comprising the two electrode segments 100 was manufactured in the same way as the electrode array 200 of the second embodiment, except that the connecting area 280 that connects the two electrode segments 100 is rectangular in shape.

### Fifth Embodiment of the Invention

The electrode array 200 of the fifth embodiment is shown in Fig. 3, and is the same as the electrode array 200 of the fourth embodiment, except that according to the fifth embodiment, connection wires (not shown) were electrically connected in parallel to the connector elements 190 (not shown in Fig. 3) in the form of a snap-fit element, such that each measuring electrode has a branch of the same electrical signal wire, which measures the same electric potential at each measuring electrode.

### Sixth Embodiment of the Invention

In the sixth embodiment of the invention, another electrode array 200 according to the invention is shown. Fig. 4A is a top view for the electrode array 200 according to the invention comprising three electrode segments 100 according to the invention. The electrode array 200 comprising the three electrode segments 100 was manufactured in the same way as the electrode array 200 of the second embodiment, except that the connecting area 280 that connects the three electrode segments 100 is "T" - shaped.

### Seventh Embodiment of the Invention

The electrode array 200 of the seventh embodiment is shown in Fig. 4A, and is the same as the electrode array 200 of the sixth embodiment, except that according to the seventh embodiment, connection wires (not shown) were electrically connected in parallel to the connector elements 190 (not shown in Fig. 4A) in the form of a snap-fit element, such that each measuring electrode has a branch of the same electrical signal wire, which measures the same electric potential at each measuring electrode.

### Eighth Embodiment of the Invention

In the eighth embodiment of the invention, another electrode array 200 according to the invention is shown. Fig. 4B is a top view for the electrode array 200 according to the invention comprising three electrode segments 100 according to the invention. The electrode array 200 comprising the three electrode segments 100 was manufactured in the same way as the electrode array 200 of the second embodiment, except that the connecting area 280 that connects the three electrode segments 100 is triangular in shape.

### Ninth Embodiment of the Invention

The electrode array 200 of the ninth embodiment is shown in Fig. 4B, and is the same as the electrode array 200 of the eighth embodiment, except that according to the ninth embodiment, connection wires (not shown) were electrically connected in parallel to the connector elements 190 (not shown in Fig. 4B) in the form of a snap-fit element, such that each measuring electrode has a branch of the same electrical signal wire, which measures the same electric potential at each measuring electrode.

The foregoing description of the presented embodiments is provided to enable a person skilled in the art to make and/or use the present invention, and is not intended to limit the scope of protection as defined by the claims.

### LIST OF REFERENCE NUMERALS

- 100: electrode segment
- 110: measuring electrode

- 140: solid space
- 145: perforated area
- 150: void space
- 160: surface-continuous area
- 190: connector element
- 195: sensor element

- 200: electrode array
- 220: mounting layer
- 221: first side of the mounting layer
- 222: other side of the mounting layer
- 230: adhesive layer
- 280: connecting area

## Claims

1. An electrode segment (100) for electrocardiographic and/or bioimpedance measurements comprising a measuring electrode (110), which comprises a connector element (190) for connecting an electrical signal wire and a sensor element (195) for collecting measurement signals from the patient's body, the measuring electrode (110) being situated in a mounting layer (220) covered with an adhesive layer (230), wherein the measuring electrode (110) situated in the mounting layer (220) is surrounded by a perforated area (145) which is free from the adhesive layer (230), and the perforated area (145) is in turn surrounded by a surface-continuous area (160) which is covered with the adhesive layer (230), wherein the perforated area (145) comprises radially arranged alternating void spaces (150) and solid spaces (140).

2. The electrode segment (100) according to claim 1, wherein the measuring electrode (110) is situated substantially centrally in the perforated area (145).

3. The electrode segment (100) according to claim 1 or 2, wherein the perforated area (145) is substantially round in shape.

4. The electrode segment (100) according to claim 1, wherein the solid spaces (140) and the void spaces (150) are elongated in shape and are arranged radially from the measuring electrode (110) to the surface-continuous area (160).

5. The electrode segment (100) according to any one of the preceding claims, wherein the surface-continuous area (160), which is covered with the adhesive layer (230) is substantially annular in shape.

6. The electrode segment (100) according to any one of the preceding claims, wherein the mounting layer (220) and the adhesive layer (230) are flexible layers configured to adapt their shape to the shape of the substrate to which they are being attached.

7. The electrode segment (100) according to any one of the preceding claims, wherein the thickness of the adhesive layer (230) is smaller than the thickness of the sensor element (195) of the measuring electrode (110).

8. The electrode segment (100) according to any one of the preceding claims, wherein the connector element (190) is a snap-fit socket for the electrical signal wire.

9. An electrode array (200) for electrocardiographic and/or bioimpedance measurements, wherein the array comprises at least two electrode segments (100) according to claims 1 to 8, and the electrode segments (100) are connected to each other by a connecting area (280) which is adjacent to the particular electrode segments (100).

10. The electrode array (200) according to claim 9, wherein the connecting area (280) forms a uniform layer with the surface-continuous area (160) of the electrode segment (100) and is covered with the adhesive layer (230).

11. The electrode array (200) according to claim 9 or 10, wherein the connecting area (280) is substantially centrally situated and surrounded by the electrode segments (100).

12. The electrode array (200) according to any one of claims 9 to 11, wherein the electrode segments (100) connected by the connecting area (280) are arranged such that each electrode segment (100) is situated substantially equidistantly from the adjacent electrode segment (100).

13. The electrode array (200) according to any one of claims 9 to 12, wherein it has a multi-segment configuration with free bending of the particular electrode segments (100) configured to adapt their shape to the shape of the substrate to which they are being adhered.

14. The electrode array (200) according to any one of claims 9 to 13, wherein the connector elements (190) of the measuring electrode (110) are electrically connected in parallel such that each measuring electrode (110) has a branch of the same electrical signal wire which measures the same electric potential at each measuring electrode (110).

## Patentansprüche

1. Ein Elektrodensegment (100) für elektrokardiographische und/oder Bioimpedanzmessungen, umfassend eine Messelektrode (110), die ein Verbindungselement (190) zum Anschließen eines elektrischen Signalkabels und ein Sensorelement (195) zum Erfassen von Messsignalen vom Körper des Patienten umfasst, wobei die Messelektrode (110) in einer Montageschicht (220) angeordnet ist, die mit einer Klebeschicht (230) bedeckt ist, wobei die in der Montageschicht (220) angeordnete Messelektrode (110) von einem perforierten Bereich (145) umgeben ist, der frei von der Klebeschicht (230) ist, und der perforierte Bereich (145) wiederum von einem oberflächenkontinuierlichen Bereich (160) umgeben ist, der mit der Klebeschicht (230) bedeckt ist, wobei der perforierte Bereich (145) radial angeordnete abwechselnde Hohlräume (150) und feste Räume (140) umfasst.

2. Elektrodensegment (100) nach Anspruch 1, wobei die Messelektrode (110) im Wesentlichen mittig im perforierten Bereich (145) angeordnet ist.

3. Elektrodensegment (100) nach Anspruch 1 oder 2, wobei der perforierte Bereich (145) im Wesentlichen rund geformt ist.

4. Elektrodensegment (100) nach Anspruch 1, wobei die festen Räume (140) und die Hohlräume (150) länglich geformt sind und radial von der Messelektrode (110) zum oberflächenkontinuierlichen Bereich (160) angeordnet sind.

5. Elektrodensegment (100) nach einem der vorhergehenden Ansprüche, wobei der oberflächenkontinuierliche Bereich (160), der mit der Klebstoffschicht (230) bedeckt ist, im Wesentlichen ringförmig geformt ist.

6. Elektrodensegment (100) nach einem der vorhergehenden Ansprüche, wobei die Befestigungsschicht (220) und die Klebeschicht (230) flexible Schichten sind, die so konfiguriert sind, dass sie ihre Form an die Form des Substrats anpassen, an dem sie angebracht werden.

7. Elektrodensegment (100) nach einem der vorhergehenden Ansprüche, wobei die Dicke der Klebeschicht (230) kleiner als die Dicke des Sensorelements (195) der Messelektrode (110) ist.

8. Elektrodensegment (100) nach einem der vorhergehenden Ansprüche, wobei das Verbindungselement (190) eine Schnappfassung für das elektrische Signalkabel ist.

9. Elektrodenanordnung (200) für elektrokardiographische und/oder Bioimpedanzmessungen, wobei die Anordnung mindestens zwei Elektrodensegmente (100) nach den Ansprüchen 1 bis 8 umfasst und die Elektrodensegmente (100) durch einen Verbindungsbereich (280) miteinander verbunden sind, der an die jeweiligen Elektrodensegmente (100) angrenzt.

10. Elektrodenanordnung (200) nach Anspruch 9, wobei der Verbindungsbereich (280) mit dem flächenkontinuierlichen Bereich (160) des Elektrodensegments (100) eine gleichmäßige Schicht bildet und mit der Klebeschicht (230) bedeckt ist.

11. Elektrodenanordnung (200) nach Anspruch 9 oder 10, wobei der Verbindungsbereich (280) im Wesentlichen zentral angeordnet und von den Elektrodensegmenten (100) umgeben ist.

12. Elektrodenanordnung (200) nach einem der Ansprüche 9 bis 11, wobei die durch den Verbindungsbereich (280) verbundenen Elektrodensegmente (100) so angeordnet sind, dass jedes Elektrodensegment (100) im Wesentlichen gleich weit vom benachbarten Elektrodensegment (100) entfernt angeordnet ist.

13. Elektrodenanordnung (200) nach einem der Ansprüche 9 bis 12, wobei sie eine mehrsegmentige Konfiguration mit freier Biegung der einzelnen Elektrodensegmente (100) aufweist, die so konfiguriert sind, dass sie ihre Form an die Form des Substrats anpassen, an das sie geklebt werden.

14. Elektrodenanordnung (200) nach einem der Ansprüche 9 bis 13, wobei die Verbindungselemente (190) der Messelektrode (110) elektrisch parallel geschaltet sind, so dass jede Messelektrode (110) einen Zweig desselben elektrischen Signaldrahts aufweist, der an jeder Messelektrode (110) dasselbe elektrische Potenzial misst.

## Revendications

1. Segment d'électrode (100) pour mesures électrocardiographiques et/ou de bioimpédance comprenant une électrode de mesure (110) qui comprend un élément connecteur (190) pour connecter un fil de signal électrique et un élément capteur (195) pour collecter des signaux de mesure provenant du corps du patient, l'électrode de mesure (110) étant située dans une couche de montage (220) recouverte d'une couche adhésive (230), l'électrode de mesure (110) située dans la couche de montage (220) étant entourée d'une zone perforée (145) qui est exempte de la couche adhésive (230), et la zone perforée (145) étant à son tour entourée d'une zone continue en surface (160) qui est recouverte de la couche adhésive (230), la zone perforée (145) comprenant des espaces vides (150) et des espaces pleins (140) alternés disposés radialement.

2. Segment d'électrode (100) selon la revendication 1, dans lequel l'électrode de mesure (110) est située approximativement au centre de la zone perforée (145).

3. Segment d'électrode (100) selon la revendication 1 ou 2, dans lequel la zone perforée (145) est de forme approximativement ronde.

4. Segment d'électrode (100) selon la revendication 1, dans lequel les espaces pleins (140) et les espaces vides (150) sont de forme allongée et sont disposés radialement de l'électrode de mesure (110) à la zone continue en surface (160).

5. Segment d'électrode (100) selon l'une quelconque des revendications précédentes, dans lequel la zone continue en surface (160), qui est recouverte de la couche adhésive (230), est de forme approximativement annulaire.

6. Segment d'électrode (100) selon l'une quelconque des revendications précédentes, dans lequel la couche de montage (220) et la couche adhésive (230) sont des couches flexibles configurées pour adapter leur forme à la forme du substrat auquel elles sont fixées.

7. Segment d'électrode (100) selon l'une quelconque des revendications précédentes, dans lequel l'épaisseur de la couche adhésive (230) est inférieure à l'épaisseur de l'élément capteur (195) de l'électrode de mesure (110).

8. Segment d'électrode (100) selon l'une quelconque des revendications précédentes, dans lequel l'élément connecteur (190) est une douille à encliquetage pour le fil de signal électrique.

9. Réseau d'électrodes (200) pour des mesures électrocardiographiques et/ou de bioimpédance, dans lequel le réseau comprend au moins deux segments d'électrode (100) selon les revendications 1 à 8, et les segments d'électrode (100) sont reliés l'un à l'autre par une zone de connexion (280) qui est adjacente aux segments d'électrode particuliers (100).

10. Réseau d'électrodes (200) selon la revendication 9, dans lequel la zone de connexion (280) forme une couche uniforme avec la zone continue en surface (160) du segment d'électrode (100) et est recouverte de la couche adhésive (230).

11. Réseau d'électrodes (200) selon la revendication 9 ou 10, dans lequel la zone de connexion (280) est située approximativement au centre et entourée par les segments d'électrode (100).

12. Réseau d'électrodes (200) selon l'une quelconque des revendications 9 à 11, dans lequel les segments d'électrode (100) reliés par la zone de connexion (280) sont disposés de telle sorte que chaque segment d'électrode (100) soit situé approximativement à égale distance du segment d'électrode adjacent (100).

13. Réseau d'électrodes (200) selon l'une quelconque des revendications 9 à 12, lequel présente une configuration à segments multiples avec une courbure libre des segments d'électrodes particuliers (100) configurés pour adapter leur forme à la forme du substrat auquel ils sont collés.

14. Réseau d'électrodes (200) selon l'une quelconque des revendications 9 à 13, dans lequel les éléments de connecteur (190) de l'électrode de mesure (110) sont connectés électriquement en parallèle de telle sorte que chaque électrode de mesure (110) présente une branche du même fil de signal électrique qui mesure le même potentiel électrique au niveau de chaque électrode de mesure (110).
